# FASCICULE DE BREVET EUROPEEN

(11) **EP 2 398 406 B1**
(45) Date de publication et mention de la délivrance du brevet: **03.04.2013**
(21) Numéro de dépôt: 10703851.5
(22) Date de dépôt: 10.02.2010
(51) Int. Cl.: A61B 17/30

(54) **PINCE CHIRURGICALE**
CHIRURGISCHE ZANGE
SURGICAL FORCEPS

(30) Priorité: 19.02.2009 FR 0900758
(43) Date de publication de la demande: 28.12.2011
(73) Titulaire: Moria SA, 92160 Antony (FR)
(72) Inventeur: AUFAURE, Jean-Luc, F-03210 Souvigny (FR); SEMPE, Antoine, F-92130 Issy les Moulineaux (FR)
(74) Mandataire: Lavialle, Bruno François Stéphane
(86) Numéro de dépôt international: PCT/EP2010/051620
(87) Numéro de publication internationale: WO 2010/094603

(56) Documents cités:
- EP-A1- 0 159 453
- EP-A2- 0 177 252
- WO-A1-2006/136663
- AU-A- 5 005 679
- US-A- 4 452 106

## Description

La présente invention concerne une pince chirurgicale et plus particulièrement une pince chirurgicale destinée à l'ophtalmologie.

### ARRIERE PLAN DE L'INVENTION

La chirurgie de l'oeil requiert d'utiliser des instruments petits et fins pour intervenir au travers d'ouvertures de petites tailles et sur des organes ou tissus également de dimensions réduites.

Certaines des pinces de ce type sont fabriquées à partir de deux lames métalliques articulées dans leur partie médiane ou au voisinage de leur extrémité active. La précision de l'extrémité active de ce type de pince dépend du soin de sa fabrication et notamment du jeu existant au niveau de l'articulation. D'ailleurs au cours de l'usage ce jeu augmente et la précision diminue.

Il existe par ailleurs des pinces qui sont fabriquées à partir de lames plates qui sont cintrées perpendiculairement à leur épaisseur et qui sont réunies par leur extrémité opposée à leur extrémité active. La raideur des bras que forment les lames n'est généralement pas satisfaisante et le praticien manque de ressenti dans la manipulation de la pince.

La réduction des coûts de fabrication de ce type de matériel est une préoccupation constante des fabricants d'autant que l'usage unique est préféré à l'usage multiple avec stérilisation après chaque usage. Il est donc apparu sur le marché des pinces de microchirurgie en matière plastique qui n'ont pas donné satisfaction en ce qui concerne la partie active de la pince par manque de finesse et de dureté. On a donc proposé des pinces composites dans lesquelles la partie active est formée de deux pointes métalliques surmoulées par une partie en matière plastique monobloc qui assure à la fois le rôle de la partie ou manche de préhension de la pince et la fonction d'écartement élastique des pointes. La plus ou moins grande souplesse de ces pinces est donnée par cette partie en plastique qui n'est pas stable dans le temps. En effet, la matière plastique subit un traitement sévère du fait des procédures de stérilisation auxquelles les pinces sont soumises qui accélèrent son vieillissement et dégradent rapidement ses qualités.

Un autre inconvénient de ce type de matériel est que la matière plastique ne permet pas de garantir un alignement parfait des pointes actives des pinces lors de leur rapprochement. Il faut donc mettre en place des moyens de centrage particuliers au niveau des parties métalliques de la pince tel que l'emboîtement d'un ergot et d'un trou ménagés chacun dans l'une des branches de la pince.

Il existe enfin des pinces dont les bras sont les deux branches d'une épingle (en forme de U) découpée dans une tôle métallique, chacun des bras étant alors équipé d'un manche ou d'une poignée sur lesquels appuient les doigts du chirurgien. L'un des intérêts de cette pince réside dans la précision de sa fabrication obtenue à faible coût. Les pointes ou becs travaillants font l'objet d'une reprise de manière à affiner leur extrémité, qu'ils soient dans le prolongement des bras, c'est-à-dire dans le plan de la tôle (pointes) ou recourbés par rapport à ceux-ci (becs), c'est--à-dire relevés par rapport au plan de cette tôle. On a constaté cependant qu'il fallait, comme pour les autres pinces connues, assurer le guidage des pointes ou bec de manière qu'ils coïncident exactement au moment du serrage.

EP-A-0159453 montre une pince comportant une pièce travaillant en forme de U formée par la réunion de deux branches séparées ayant chacune une extrémité libre conformée en pointe. La pièce travaillante a une épaisseur dont la dimension est parallèle au plan de débattement des branches.

Dans les pinces obtenues par découpe, il n'existe pas suffisamment de matière métallique dans chaque branche pour procéder de la sorte.

Par la présente invention, on entend proposer une pince comportant une épingle métallique obtenue soit par découpe d'une tôle soit par la technique du moulage par injection métallique (M.I.M.), équipée de manière simple de moyens pour assurer une parfaite coaptation des pointes travaillantes lors de la manipulation de la pince par le chirurgien.

### OBJET DE L'INVENTION

La présente invention a donc pour objet une pince pour chirurgie ophtalmologique comportant d'une part une pièce travaillante monobloc, en forme de U, dont l'extrémité libre de chacune des branches est conformée en une pointe, la pièce étant issue d'une ébauche plate dont l'épaisseur est la dimension perpendiculaire au plan de débattement des branches et d'autre part des éléments de manoeuvre de la pièce travaillante formant ensemble un manche de préhension de la pince caractérisée en ce que chaque élément est en forme de corps allongé présentant une surface extérieure convexe et une surface sensiblement plane creusée d'une rainure longitudinale de logement d'au moins une des branches de la pièce travaillante, l'extrémité de chaque élément du manche tournée du côté de la pointe étant pourvue de moyens de centrage coopérant avec un organe de centrage complémentaire de l'extrémité correspondante de l'autre élément.

On a en effet constaté que la distorsion de la pince a pour origine la direction de l'effort qu'elle reçoit de la part des doigts du chirurgien. En effet cet effort peut ne pas être strictement contenu dans le plan de débattement des branches du U mais peut être incliné par rapport à ce plan, créant ainsi une composante transversale s'ajoutant à l'effort de pincement, qui conduit à un gauchissement du plan de la pièce travaillante et donc à l'écartement latéral des pointes. Les moyens de centrage de l'invention viennent s'opposer à ce gauchissement en encaissant cette composante latérale de l'effort de serrage.

Dans un mode préféré de réalisation de l'invention, ces moyens de centrage sont constitués par au moins des ailes prévues sur l'un des corps, faisant saillie vers l'autre corps, au delà de la surface plane rainurée pour encadrer de manière ajustée une portion complémentaire de l'extrémité de l'autre corps lors du pincement.

Les ailes en question seront avantageusement divergentes à partir de la surface plane tandis que la portion complémentaire présentera la forme d'une cale à faces convergentes venant progressivement se loger entre les ailes dans le mouvement de pincement.

Selon une particularité de l'invention, chaque branche est pourvue en regard de l'autre d'une saillie constituant une butée d'arrêt du pincement, cette butée étant située au niveau des moyens de centrage susdits.

Les deux éléments du manche de la pince sont d'une longueur inférieure à la longueur totale de la pièce travaillante. Chacun est alors rapporté sur la branche correspondante par emboîtement de celle-ci dans la rainure et fixation par tout moyen approprié aux matériaux constituant et la pièce travaillante et le manche (soudure au laser s'il s'agit d'un matériau métallique pour les deux, collage, encliquetage, ....). Dans ce cas, la pièce travaillante possède une partie de racine des branches s'étendant très au-delà de l'extrémité arrière (proximale) des éléments du manche de manière à aider à la manipulation de la pince par l'opérateur.

Dans une variante de réalisation, les éléments du manche se prolongent au-delà de cette extrémité proximale de la pièce travaillante et sont réunies par leur extrémité extérieure à cette pièce travaillante.

Selon une caractéristique importante de l'invention, chaque branche de la pièce travaillante est issue d'une partie de racine formant la base du U par le biais d'une zone à largeur contrôlée. Le contrôle, à la fabrication de la largeur de cette zone, permet d'adapter la raideur de la pince c'est-à-dire sa résistance à l'effort de pincement. En effet, selon l'usage de la pince, la raideur de la pince soit être plus ou moins importante (en général plus importante lors d'une opération requérant une précision).

On mentionnera en outre une réalisation particulière de l'invention dans laquelle les éléments de manche sont en matière plastique injectée caractérisée en ce que chaque élément comporte une rainure dont au moins deux zones sont calibrées en largeur, le fond de la rainure comportant une ouverture destinée à recevoir une dent d'accrochage inclinée externe d'une branche de la pince, l'extrémité proximale de cette rainure étant apte à immobiliser l'élément de manche sur la pièce travaillante en coopération avec la dent tandis que l'extrémité de cet élément de manche est conformé en une queue plate sensiblement perpendiculaire au plan de la pièce travaillante, cette queue étant pourvue de moyens d'encliquetage par pression coopérant avec des moyens complémentaires prévus au niveau de la queue de l'autre élément de manche.

Selon une autre caractéristique de l'invention réalisée comme ci-dessus avec des éléments de manche séparés en matière plastique, au moins l'une ou l'autre des ailes ou portions complémentaires comporte des reliefs sur au moins l'une des faces actives de sorte qu'au premier rapprochement des branches de la pince, les reliefs soient au moins partiellement plastiquement écrasés.

Cette disposition permet d'obtenir une excellente coaptation des pointes ou becs de la pince, malgré les incertitudes de dimensions des pièces en matière plastique.

Enfin, pour maîtriser l'interaction entre la partie travaillante et le manche en plastique, c'est-à-dire pour que les efforts de l'un sur l'autre soient de nature à assurer le maintien correct des éléments de manche sur la partie travaillante avec un équilibre des contraintes de manière à ne pas engendrer de distorsions altérant les qualités de la coaptation ou conduisant à leur dégradation au cours du temps par suite des stérilisations successives, l'un des flancs de chaque rainure est pourvu d'au moins un relief plastiquement déformable par l'introduction de la pièce travaillante dans la rainure.

D'autres caractéristiques et avantages de l'invention apparaîtront à la lecture de la description donnée ci-après de quelques modes de réalisation. Il sera fait référence aux dessins annexés parmi lesquels :

### BREVE DESCRIPTION DES DESSINS

- la figure 1 illustre par une vue extérieure l'extrémité distale d'une pince de microchirurgie conforme à l'invention,
- la figure 2 est une vue en coupe de cette pince,
- la figure 3 illustre par une vue en perspective une pièce travaillante dans laquelle la pince est en forme de bec,
- la figure 4 est une vue en coupe longitudinale d'une deuxième réalisation de l'invention,
- la figure 5 est une vue en plan d'un élément de manche d'une pince selon la figure 4,
- la figure 6 illustre l'extrémité d'une variante de réalisation de la pince selon les figures 4 et 6,
- la figure 7 est une vue de détail d'un élément de manche en matière plastique de la pince selon l'invention.

### DESCRIPTION DETAILLEE DE L'INVENTION

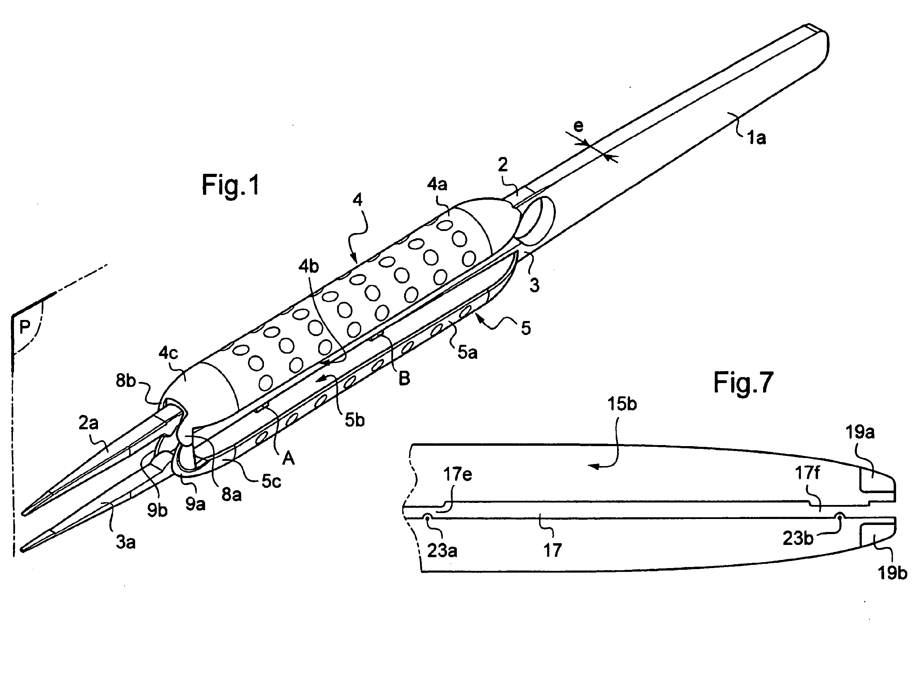

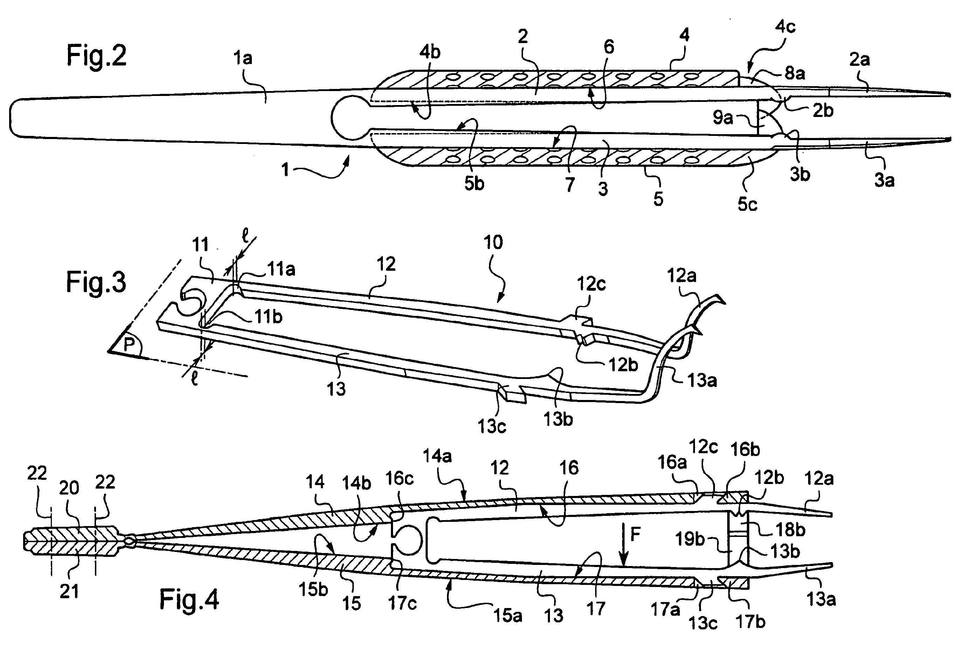

Aux figures 1 et 2 la pince pour chirurgie ophtalmologique représentée comporte une pièce travaillante monobloc 1, en forme de U, issue d'une tôle métallique par découpage au fil (électroérosion) ou toute autre technique comme l'usinage électrochimique de précision (PECM). Cette pièce 1 comporte donc deux branches 2 et 3 dont l'extrémité libre 2a, 3a de chacune d'elles est conformée en une pointe, notamment par usinage. L'épaisseur e de la tôle (ou d'une manière générale, d'une ébauche plate qui pourrait être une plaque de matériau synthétique, soit du type à matrice de polymère chargée d'une matière de renforcement, soit du type composite soit issue du procédé de fabrication MIM (moulage par injection métallique)) est la dimension perpendiculaire au plan P de débattement des branches.

La pince comporte aussi des éléments 4 et 5 de manoeuvre de la pièce travaillante 1, formant ensemble un manche de préhension de la pince.

Chaque élément 4, 5 est en forme de corps allongé présentant une surface extérieure 4a, 5a convexe et une surface sensiblement plane 4b, 5b creusée d'une rainure longitudinale 6, 7 de logement d'une branche 2,3 de la pièce travaillante 1. La surface convexe constitue la surface de grip de la pince et sera pourvue de toute texturation ou revêtement utile au confort et à la qualité de la préhension.

L'extrémité 4c, 5c de chaque élément du manche qui est tournée du côté de la pointe 2a, 3a est pourvue d'un organe de centrage coopérant avec un organe de centrage complémentaire de l'extrémité correspondante de l'autre élément. Dans le cas des figures 1 et 2, ces moyens de centrage sont constitués par au moins des ailes 8a, 8b prévues sur le corps 4 et 9a, 9b prévues sur le corps 5, faisant saillie vers l'autre corps, au delà de la surface plane rainurée 4a, 5a du corps correspondant. Les ailes 8a, 8b du corps 4 encadrent de manière ajustée les ailes 9a, 9b portées par l'autre corps 5. Ainsi, leur coulissement à très faible jeu et leur recouvrement partiel même au repos comme illustré aux figures 1 et 2, empêche tout gauchissement de la pince malgré l'existence d'une composante transversale de l'effort exercé sur la pince pour la refermer et conserve l'alignement des pointes jusqu'à leur contact. Dans une variante de réalisation, la surface extérieure des ailes 9a et 9b peut converger vers l'autre en direction du corps 4 alors que les surfaces intérieures des ailes 8a et 8b divergent l'une de l'autre en direction du corps 5 ; l'effet de came résultant de cette géométrie permet le ré-alignement progressif des pointes au fur et à mesure du serrage de la pince.

On notera que chaque branche de la pince présente en regard de l'autre une saillie 2b, 3b, constituant une butée qui limite le rapprochement des deux branches afin d'éviter un serrage excessif qui conduirait à la réouverture des pointes. Cette butée est située au niveau des moyens de centrage 8a, 8b, 9a, 9b.

Dans le cas des figures 1 et 2, le manche est plus court que la pince, c'est-à-dire que la pièce travaillante 1 est plus longue que les éléments 4 et 5. Ces éléments sont ici portés par les branches de la pince 1 qui possède une partie la de racine de ces branches dont la longueur est pratiquement celle des branches. En ayant choisi une longueur appropriée pour la partie de racine la de la pièce 1, on peut compenser le déséquilibre vers l'avant du au poids du manche.

La fixation des éléments 4 et 5 sur les branches 2 et 3 dépend de la nature des matériaux à assembler. Quand les deux matériaux sont métalliques, on peut assurer cette fixation par soudure au laser comme illustré par les points A et B de la figure 1. Dans d'autres cas, la fixation sera assurée par collage ou soudure par ultrasons,....

Le mode de réalisation de la pince illustré aux figures suivantes 3 à 5, comprend une pièce travaillante 10 d'un dessin différent de celui de la pièce 1 précédemment décrite. Les deux branches 12 et 13 de cette pièce sont issues d'une partie de racine 11 beaucoup plus courte que la précédente. L'extrémité libre 12a, 13a, de chaque branche est pourvue de pointes en forme de bec qui s'étendent à l'extérieur du plan P de débattement des branches. Le raccordement entre les branches 12 et 13 et la partie de racine 11 est réalisé au travers de zones 11a et 11b dont la largeur 1 est définie par usinage et détermine la raideur élastique de la pince. Ainsi, à partir d'une même ébauche peut-on fabriquer des pinces de qualités différentes pour des applications différentes pour satisfaire toutes les demandes des chirurgiens.

Chaque branche possède une saillie 12b 13b, de limitation du mouvement de serrage de la pince, ces saillies étant ici de forme complémentaire (une pointe pour la saillie 13b et un V pour la saillie 12b). Au voisinage de cette butée, chaque branche comporte un ergot extérieur 12c, 13c qui constitue une dent inclinée vers la pointe.

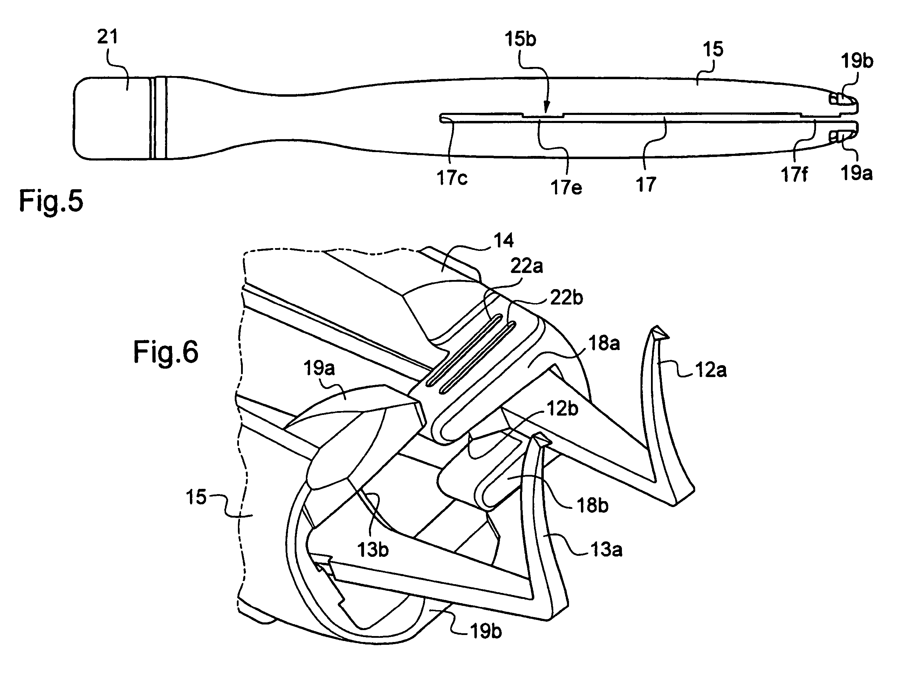

A la figure 4, la pièce 10 est représentée montée dans un manche qui comporte deux éléments 14 et 15. La figure 5 est une vue en plan suivant F de la figure 4 de l'élément 15.

Chacun des éléments 14 et 15 est de forme allongée pour présenter comme dans la pince précédente, une surface d'extrados convexe 14a, 15a, une surface d'intrados sensiblement plane 14b, 15b avec une rainure 16, 17. Le fond de chaque rainure comporte une ouverture 16a, 17a destinée à recevoir la dent inclinée 12c, 13c externe de chaque branche 12, 13 de la pièce travaillante 10. Les ouvertures 16a, 17a sont également inclinées vers l'avant c'est-à-dire que l'extrémité distale de ces ouvertures forme une dent 16b, 17b complémentaire de la dent 12c, 13c correspondante de la pièce travaillante lorsqu'elle est logée dans la rainure 16, 17.

L'autre extrémité 16c, 17c (proximale) de cette rainure 16, 17 est apte à immobiliser longitudinalement l'élément de manche sur la pièce travaillante en coopération avec la dent correspondante. Pour cela, la longueur de la rainure c'est-à-dire la dimension qui sépare l'ouverture 16a, 17a de l'extrémité correspondante 16c, 17c est comprise dans des tolérances qui ne permettent pas, une fois la pièce 10 logée dans chaque rainure d'en échapper par un simple glissement longitudinal relatif par rapport au manche.

Chacun des éléments 14, 15 du manche est pourvue à son extrémité proche des pointes de la pince, de moyens de centrage 18b, 19a, 19b semblables à ceux décrits précédemment. L'autre extrémité de chaque élément de manche est conformée en une queue plate 20, 21 sensiblement perpendiculaire au plan P de la pièce travaillante 10 (et de son débattement), cette queue 20 étant pourvue de moyens d'encliquetage 22 par pression coopérant avec des moyens complémentaires prévus à la queue 21 de l'autre élément de manche. Ces moyens d'encliquetage ne sont représentés que symboliquement, car ils peuvent prendre de nombreuses formes appropriées connues en elles mêmes (encliquetages définitifs à dents de sapin ou encliquetages démontables à boutons pressions,...). Cette variante de réalisation permet de prévoir une possibilité de fabriquer des manches selon une standardisation déterminée pouvant accepter plusieurs types de pièces travaillantes. En outre, si l'encliquetage est démontable, la pince de l'invention présente une grande facilité de recyclage.

En référence plus particulière à la figure 5, on constate qu'une rainure telle que 17, comporte des zones de largeur calibrée 17e, 17f d'étendue restreinte pour permettre une bonne maîtrise de cette largeur par un procédé de fabrication par injection. Cette largeur correspond à l'épaisseur e de la plaque de laquelle est issue l'ébauche de la pièce travaillante. On comprend que seules deux zones précises sont déterminantes pour cette dimension e de la pièce travaillante et qu'ainsi, si la plaque de départ est d'épaisseur par trop variable, une simple passe de surfaçage n'est nécessaire que localement, sur des régions de la plaque devant être logées dans ces zones à largeur calibrée.

La figure 6 est une vue en perspective d'une extrémité d'une variante de réalisation de la pince représentée aux figures 3 et 4.

Sur cette figure, on retrouve les éléments déjà décrits avec les mêmes références.

Ainsi, on retrouve à l'extrémité des éléments de manche 14 et 15 voisines des pointes 12a et 13a de la pince, les moyens de centrage 18a, 18b et 19a, 19b. Plus précisément les ailes 19a, 19b prévues à l'extrémité du corps 15 sont disposées de manière à encadrer les portions 18a et 18b de l'élément 14 en protubérance en regard des ailes. Les protubérances 18a, 18b guident les ailes divergentes à la manière d'un coin lorsque celles-ci viennent les encadrer lors de la fermeture de la pince sous les doigts de l'opérateur. Leurs faces extérieures convergent vers la branche opposée de la pince tandis que les faces intérieures des ailes 19a, 19b divergent en direction de la branche opposée. Le centrage est réalisé par coïncidence de ces faces externes et internes. Il est possible que dans certains cas le centrage n'ait pas eu lieu avant le contact des pointes, compte tenu des incertitudes dimensionnelles de la fabrication des éléments en plastique et de celles résultant du montage des éléments 14 et 15 sur la partie travaillante de la pince. Il s'agit là d'un inconvénient car lors de chaque fermeture de pince, la coïncidence des pointes devient plus hasardeuse. En effet, l'effort appliqué par le chirurgien sur le manche de la pince peut induire de légers efforts de couple qui tendent à créer une torsion de la pièce métallique qui forme la partie active de la pince et un décalage des extrémités 12a, 13a l'une par rapport à l'autre qui ne sera pas contré totalement par les moyens de centrage.

Selon la variante représentée, pour remédier à cet inconvénient, on prévoit une disposition qui vient en quelque sorte garantir le contact et donc le centrage des éléments 18a, 18b avec ceux 19a, 19b de l'autre branche.

Cette disposition consiste en deux reliefs 22a, 22b ménagés sur la face externe d'au moins une et de préférence des deux protubérances 18a, 18b. Ces reliefs, venus d'une seule pièce avec la matière du manche 14, ont une dimension telle que d'une part, leur contact avec la face interne des ailes 19a et 19b intervient avant la fermeture totale de la pince et que d'autre part, lorsqu'après le contact, la fermeture se poursuit, il se produit un écrasement de ces reliefs. Ces reliefs écrasés seront alors la surface de contact et donc de centrage des branches de la pince l'une par rapport à l'autre, qui sera exactement conformée pour obtenir une coïncidence parfaite des pointes de la pince 12a, 13a, pourvu que lors de la première fermeture l'opérateur ait pris soin de veiller à la réalisation de cette coïncidence.

Bien entendu, ces reliefs peuvent être réalisés sur la face interne des ailes 19a, 19b ou partagés entre les surfaces destinées au guidage de la fermeture de la pince.

On se référera enfin à la figure 7 où ont été représentés des reliefs 23a et 23b, qui sont également destinés à être au moins partiellement écrasés quand la partie active 10 métallique de la pince est logée dans la rainure 14 de chaque élément de manche 14 et 15. On est ainsi certain du fait que le logement de cette partie ne « flotte » pas latéralement dans les éléments de manche. Ces reliefs seront de préférence situés dans les zones 17c et 17f de la fente, zones où la dimension transversale est la mieux maîtrisée.

## Revendications

1. Pince pour microchirurgie notamment ophtalmologique comportant une pièce travaillante monobloc (1,10), en forme de U, dont l'extrémité libre (2a, 3a, 12a, 13a) de chacune des branches (2,3,12,13) est conformée en une pointe, la pièce étant issue d'une ébauche plate dont l'épaisseur (e) est la dimension perpendiculaire au plan (P) de débattement des branches (2,3,12,13) et des éléments de manoeuvre de la pièce travaillante formant ensemble un manche de préhension de la pince **caractérisée en ce que** chaque élément est en forme de corps allongé (4,5,14,15) présentant une surface extérieure convexe (4a,5a,14a,15a) et une surface sensiblement plane (9b,5b, 19b,15b) creusée d'une rainure (6,7,16,17) longitudinale de logement d'une des branches (2,3,12,13) de la pièce travaillante (1,10), l'extrémité de chaque élément du manche tournée du côté de la pointe étant pourvue de moyens de centrage (8a,8b,9a,9b,18b,19a,19b) coopérant avec un organe de centrage complémentaire de l'extrémité correspondante de l'autre élément.

2. Pince selon la revendication 1, **caractérisée en ce que** les moyens de centrage sont constitués par au moins des ailes (8a,8b,9a,9b,18b,19a,19b) prévues sur l'un des corps (4,5,14,15), faisant saillie vers l'autre corps, au delà de la surface plane (4b,5b,14b,15b) rainurée (6,7,16,17) pour encadrer de manière ajustée une portion complémentaire (8a,8b,9a, 9b,18b,19a,19b) de l'extrémité de l'autre corps lors du pincement.

3. Pince selon la revendication 1 ou la revendication 2, **caractérisée en ce que** les ailes (9a,9b,19a,19b) seront avantageusement divergentes à partir de la surface plane tandis que la portion complémentaire (8a,8b,18b) présente la forme d'une cale à double pente venant progressivement se loger entre les ailes dans le mouvement de pincement.

4. Pince selon l'une des revendications précédentes, **caractérisée en ce que** chaque branche (2,3,12,13) est pourvue en regard de l'autre d'une saillie (2b,3b,12b,13b) constituant une butée d'arrêt du pincement, cette butée étant située au niveau des moyens de centrage susdits.

5. Pince selon l'une des revendications précédentes, **caractérisée en ce que** les deux éléments (4,5) du manche de la pince sont d'une longueur inférieure à la longueur totale de la pièce travaillante (1), chacun est alors rapporté sur la branche (2,3) correspondante par emboîtement de celle-ci dans la rainure (6,7) et fixation par tout moyen approprié aux matériaux constituant et la pièce travaillante et le manche.

6. Pince selon la revendication 5, **caractérisée en ce que** la pièce travaillante (1) possède une partie de racine (1a) des branches (2,3) s'étendant très au-delà de l'extrémité arrière (proximale) des éléments (4,5) du manche de manière à aider à la manipulation de la pince par l'opérateur.

7. Pince selon l'une des revendications 1 à 4, **caractérisée en ce que** les éléments de manche (14,15) sont en matière plastique injectée, **en ce que** chaque élément comporte une rainure (16,17) dont le fond comporte une ouverture (16a,17a) destinée à recevoir une dent (12c,13c) d'accrochage inclinée, externe d'une branche (12,13) de la pince, l'extrémité (16c,17c) proximale de cette rainure étant apte à immobiliser l'élément de manche (14,15) sur la pièce travaillante (10) en coopération avec la dent tandis que l'extrémité de cet élément de manche est conformé en une queue plate (20,21) sensiblement perpendiculaire au plan (P) de la pièce travaillante en service, cette queue étant pourvue de moyens d'encliquetage (22) par pression coopérant avec des moyens complémentaires prévus à la queue de l'autre élément de manche.

8. Pince selon la revendication 7, **caractérisée en ce qu'**au moins l'une ou l'autre des ailes (19a,19b) ou portions complémentaires (18a,18b) comporte des reliefs (22a,22b) sur au moins l'une des faces actives de sorte qu'au premier rapprochement des branches de la pince, les reliefs (22a, 22b) soient au moins partiellement plastiquement écrasés.

9. Pince selon la revendication 7 ou la revendication 8, **caractérisée en ce que** l'un des flancs de chaque rainure (17) est pourvu d'au moins un relief (23a,23b) plastiquement déformable par l'introduction de la pièce travaillante (10) dans la rainure.

10. Pince selon l'une des revendications précédentes, **caractérisée en ce que** la zone de raccordement (11a,11b) des branches (12,13) de la partie travaillante à la partie de racine (11) de ces branches est de largeur 1 contrôlée.

## Claims

1. Tweezers for microsurgery, in particular ophthalmological surgery, the tweezers comprising a one-piece working part (1, 10) of U-shape, with the free ends (2a, 3a, 12a, 13a) of each branch (2, 3, 12, 13) being shaped into a point, the part being derived from a flat blank of thickness (e) constituting the dimension perpendicular to the plane (P) in which the branches (2, 3, 12, 13) move, and elements for manipulating the working part together forming a handle for gripping the tweezers, **characterized in that** each element is in the form of an elongate body (4, 5, 14, 15) presenting a convex outside surface (4a, 5a, 14a, 15a) and a substantially plane surface (4b, 5b, 14b, 15b) having a longitudinal groove (6, 7, 16, 17) hollowed out therein to house one of the branches (2, 3, 12, 13) of the working part (1, 10), the end of each handle element facing towards the points being provided with centering means (8a, 8b, 9a, 9b, 18b, 19a, 19b) co-operating with a complementary centering member of the corresponding end of the other element.

2. Tweezers according to claim 1, **characterized in that** the centering means are constituted at least by fins (8a, 8b, 9a, 9b, 18b, 19a, 19b) provided on one of the bodies (4, 5, 14, 15) and projecting towards the other body beyond the plane surface (4b, 5b, 14b, 15b) having the groove (6, 7, 16, 17) so as to fit closely on either side of a complementary portion (8a, 8b, 9a, 9b, 18b, 19a, 19b) at the end of the other body during closure of the tweezers.

3. Tweezers according to claim 1, **characterized in that** the fins (9a, 9b, 19a, 19b) advantageously diverge away from the plane surface while the complementary portion (8a, 8b, 18b) presents the shape of a wedge having two slopes that becomes progressively received between the fins during the closure movement of the tweezers.

4. Tweezers according to claim 1, **characterized in that** each branch (2, 3, 12, 13) is provided, facing the other branch, with a projection (2b, 3b, 12b, 13b) constituting an abutment for stopping closure, said abutment being situated level with the above-mentioned centering means.

5. Tweezers according to claim 1, **characterized in that** the two handle elements (4, 5) of the tweezers are of length shorter than the total length of the working part (1), each then being fitted on the corresponding branch (2, 3) by the branch being engaged in the groove (6, 7) and by fastening by any means appropriate for the materials constituting the working part and the handle.

6. Tweezers according to claim 5, **characterized in that** the working part (1) possesses a root portion of the branches (2, 3) extending well beyond the rear ends (proximal ends) of the handle elements (4, 5) so as to facilitate handling of the tweezers by the operator.

7. Tweezers according to claim 1, **characterized in that** the handle elements (14, 15) are of injected plastics material, and **in that** each element includes a groove (16, 17) with a bottom that includes an opening (16a, 17a) for receiving an inclined connection tooth (12c, 13c) on the outside of a branch (12, 13) of the tweezers, the proximal end (16c, 17c) of the groove being suitable, in co-operation with the tooth, for preventing the handle element (14, 15) moving on the working part (10), while the end of said handle element is shaped as a flat tail (20, 21) substantially perpendicular to the plane (P) of the working part in service, said tail being provided with pressure-operated snap-fastener means (22) co-operating with complementary means provided on the tail of the other handle element.

8. Tweezers according to claim 7, **characterized in that** at least one or the other of the fins (19a, 19b) or of the complementary portions (18a, 18b) includes portions in relief (22a, 22b) on at least one of its active faces such that on the first occasion the branches of the tweezers are moved towards each other, the portions in relief (22a, 22b) are flattened, at least in part, by plastic deformation.

9. Tweezers according to claim 7, **characterized in that** one of the flanks of each groove (17) is provided with at least one portion in relief (23a, 23b) that is suitable for being deformed plastically by inserting the working part (10) in the groove.

10. Tweezers according to claim 1, **characterized in that** the zones (11a, 11b) connecting the root portion (11) of the branches to the branches (12, 13) of the working part are of controlled width ℓ.

## Patentansprüche

1. Zange zur Anwendung in der Mikrochirurgie, insbesondere in der ophtalmologischen Mikrochirurgie, umfassend ein U-förmiges, einstückiges Arbeitsstück (1, 10), bei dem das freie Ende (2a, 3a, 12a, 13a) eines jeden Schenkels (2, 3, 12, 13) als eine Spitze ausgebildet ist, wobei das Arbeitsstück aus einem flachen Rohling hervorgeht, dessen Dicke (e) die Abmessung senkrecht zu der Ebene (P) der Auslenkung der Schenkel (2, 3, 12, 13) und der Elemente zum Manövrieren des Arbeitsstückes ist, die zusammen einen Griff zum Greifen der Zange bilden, **dadurch gekennzeichnet, dass** jedes Element die Form eines länglichen Körpers (4, 5, 14, 15) hat, der eine konvexe Außenfläche (4a, 5a, 14a, 15a) sowie eine im Wesentlichen ebene Oberfläche (4b, 5b, 14b, 15b) aufweist, die mit einer Längsnut (6, 7, 16, 17) versehen ist, um einen der Schenkel (2, 3, 12, 13) des Arbeitsstücks (1, 10) aufzunehmen, wobei das Ende eines jeden Griffelements, das zur Seite der Spitze weist, mit Zentriermitteln (8a, 8b, 9a, 9b, 18b, 19a, 19b) ausgestattet ist, die mit einem komplementären Zentrierorgan des entsprechenden Endes des anderen Elements zusammenwirken.

2. Zange nach Anspruch 1, **dadurch gekennzeichnet, dass** die Zentriermittel mindestens durch Flügel (8a, 8b, 9a, 9b, 18b, 19a, 19b) gebildet sind, die an einem der Körper (4, 5, 14, 15) vorgesehen sind, und die in Richtung des anderen Körpers über die ebene Oberfläche (4b, 5b, 14b, 15b), die mit einer Nut (6, 7, 16, 17) versehen ist, überstehen, um einen komplementären Abschnitt (8a, 8b, 9a, 18b, 19a, 19b) des Endes des anderen Körpers während des Zusammendrückens passend einzufassen.

3. Zange nach Anspruch 1 oder 2, **dadurch gekennzeichnet, dass** die Flügel (9a, 9b, 19a, 19b) ab der ebenen Oberfläche vorteilhaft divergierend sind, während der komplementäre Abschnitt (8a, 8b, 18b) die Form eines doppelt abgeschrägten Keils aufweist, der bei Bewegung der Zange schrittweise zwischen die Flügel eingreift.

4. Zange nach einem der vorhergehenden Ansprüche, **dadurch gekennzeichnet, dass** die gegenüberliegenden Schenkel (2, 3, 12, 13) einen Vorsprung aufweisen (2b, 3b, 12b, 13b), der einen Stoppanschlag beim Zusammendrücken bildet, wobei sich dieser Anschlag auf Höhe der oben genannten Zentriermittel befindet.

5. Zange nach einem der vorhergehenden Ansprüche, **dadurch gekennzeichnet, dass** die beiden Griffelemente (4, 5) der Zange kürzer sind als die Gesamtlänge des Arbeitsstücks (1), jedes Element also am entsprechenden Schenkel (2, 3) durch dessen Eingriff in die Nut (6, 7) und durch Befestigung durch Mittel mantiert ist, die für das Arbeitsstück und den Griff bildenden Materialien geeignet sind.

6. Zange nach Anspruch 5, **dadurch gekennzeichnet, dass** das Arbeitsstück (1) einen Fußabschnitt (1a) der Schenkel (2, 3) besitzt, der sich weit über das hintere (proximale) Ende der Griffelemente (4, 5) hinaus erstreckt, so dass die Handhabung der Zange für den Operateur erleichtert wird.

7. Zange nach einem der Ansprüche 1 bis 4, **dadurch gekennzeichnet, dass** die Griffelemente (14, 15) aus Kunststoffspritzguss bestehen, und dass jedes Element eine Nut (16, 17) hat, der Boden eine Öffnung (16a, 17a) zur Aufnahme eines an der Außenseite eines Zangenschenkels (12, 13) vorgesehenen schrägen Haltezahnes (12c, 13c) aufweist, wobei das proximale Ende (16c, 17c) dieser Nut geeignet ist, das Griffelement (14, 15) an dem Arbeitsstück (10) durch Zusammenwirken mit dem Zahn zu immobilisieren, während das Ende dieses Griffelements als flaches Ende (20, 21) ausgebildet ist, das in Gebrauch im Wesentlichen rechtwinklig zur Ebene (P) des Arbeitsstückes ist, wobei dieses Ende mit Rastmitteln (22) ausgestattet ist, die durch Druck mit den am Ende des anderen Griffelements angebrachten komplementären Mitteln zusammenwirken.

8. Zange nach Anspruch 7, **dadurch gekennzeichnet, dass** mindestens einer der Flügel (19a, 19b) oder der komplementären Abschnitte (18a, 18b) mindestens auf einer aktiven Fläche Profile (22a, 22b) aufweist, so dass die Profile (22a, 22b) bei einer ersten Annäherung der Schenkel der Zange mindestens teilweise plastisch zusammengedrückt werden.

9. Zange nach Anspruch 7 oder Anspruch 8, **dadurch gekennzeichnet, dass** eine der Flanken einer jeden Nut (17) mit mindestens einem Profil (23a, 23b) versehen ist, das durch Einführen des Arbeitsstücks (10) in die Nut plastisch verformbar ist.

10. Zange nach einem der vorhergehenden Ansprüche, **dadurch gekennzeichnet, dass** der Verbindungsbereich (11a, 11b) der Schenkel (12, 13) des Arbeitsstückes am Fußabschnitt (11) dieser Schenkel eine kontrollierte Größe 1 besitzt.
